# EUROPEAN PATENT SPECIFICATION

(11) **EP 2 763 381 B1**
(45) Date of publication and mention of the grant of the patent: **05.04.2017**
(21) Application number: 14152288.8
(22) Date of filing: 23.01.2014
(51) Int. Cl.: H04M 1/21, G01N 33/00, G01N 33/497

(54) **Portable electronic device with chemical sensor**
Tragbare elektronische Vorrichtung mit chemischem Sensor
Dispositif électronique portable avec capteur chimique

(30) Priority: 31.01.2013 EP 13405011
(43) Date of publication of application: 06.08.2014
(73) Proprietor: Sensirion AG, 8712 Stäfa (CH)
(72) Inventor: Mayer, Felix, 8712 Stäfa (CH); Bürgi, Lukas, 8049 Zürich (CH); Graf, Markus, 8005 Zürich (CH); Röck, Frank, 9500 Wil (CH)
(74) Representative: Detken, Andreas

(56) References cited:
- WO-A1-2009/007842
- US-A- 4 749 553
- US-A1- 2008 170 762
- US-A1- 2011 308 297

## Description

### TECHNICAL FIELD

The present invention relates to a portable electronic device comprising at least one chemical sensor, and to a method of operating a portable electronic device.

### PRIOR ART

Portable electronic devices, such as mobile phones or tablet computers, are usually equipped with several sensors, e.g. a microphone, a touch screen, one or more gyroscopes, one or more acceleration sensors, a magnetic field sensor, a proximity sensor, and one or more cameras. US 2012/0231841 discloses a mobile phone equipped with a humidity sensor. WO 2012/097460 A1 suggests providing a mobile phone with a temperature sensor and a humidity sensor.

It is also possible to integrate sensors that are sensitive to one or more chemical analytes into a portable electronic device. Sensors that are sensitive to one or more chemical analytes will in the following be called "chemical sensors". US 8,280,436 discloses a breath analysis sensor in a mobile phone to determine a measure for the blood alcohol level of a user of the phone.

When measurements are carried out with a chemical sensor in a portable electronic device, it is often necessary that the user handles the portable electronic device in a specific manner. Otherwise, the results obtained from the sensor might be meaningless. For instance, if blood alcohol level is to be determined from breath, it must be ensured that the user exhales at the portable electronic device in a specific location, with sufficient strength and over a sufficient amount of time to ensure that a sufficient amount of exhaled air reaches the sensor.

Compliance with the required handling conditions can be ensured by operating the chemical sensor only if certain preconditions are fulfilled. For instance, in US 8,280,436 breath analysis is carried out only during time intervals when a phone call is made by the user. Otherwise, no measurements are carried out. However, it is often desired to carry out a measurement upon a user request, e.g., when the user pushes a button on the device, even if such preconditions are not fulfilled. In such cases other ways must be found to ensure that the portable electronic device is handled correctly.

US 4,749,553 discloses a breath alcohol detector that compensates for distances between the mouth of the individual exhaling breath into the ambient air and the detector, the atmospheric pressure, and the temperature. To this end, the breath alcohol detector comprises a distance sensor, a pressure sensor and a temperature sensor. An audible warning may be issued to instruct the individual to repeat the test by moving closer to the detector and counting while the test progresses.

US 2011/0308297 A1 discloses a breath alcohol detector that determines breath pressure. Breath pressure is displayed on a breath pressure display. The individual who is blowing can thereby recognize whether the breath pressure is appropriate or not.

WO 2009/007842 A1 discloses a breath alcohol detector comprising a breath temperature detection section or a mouth detection device with a camera, which serve as an exhalation manner detection means for detecting a manner in which the subject exhales the breath.

### SUMMARY OF THE INVENTION

In a first aspect, it is an object of the present invention to provide a portable electronic device that is equipped with at least one chemical sensor, wherein user compliance with the required handling conditions is improved when a measurement is taken with the chemical sensor, thereby improving the reliability of a result of the measurement.

This object is achieved by a portable electronic device with the features of claim 1. Further embodiments of the invention are laid down in the dependent claims.

In another aspect, it is an object of the present invention to provide a corresponding process of operating a portable electronic device.

This object is achieved by a process as laid down in claim 5.

The present invention provides a portable electronic device, comprising:
a control device;
at least one first sensor, the first sensor being sensitive to at least one chemical analyte (i.e., the first sensor is a "chemical sensor"), the first sensor being coupled to the control device; and
at least one output device coupled to the control device;
wherein the control device is configured to carry out the following steps:
carrying out a measurement with the first sensor;
determining whether the portable electronic device has been handled appropriately during the measurement; and
operating the output device to present information to a user of the portable electronic device if the portable electronic device has not been handled appropriately.

In this manner, the portable electronic device provides real-time feedback to the user whether the user has handled the device in a predetermined desired manner. This helps to ensure that meaningful results are obtained by the sensor.

Determination whether the portable electronic device has been handled appropriately during the measurement may involve determining corresponding status data, i.e. a data structure such as a corresponding indicator value, which may be a binary value or a more complicated structure, e.g., a number or a set of numbers indicating the extent to which the device has been handled accordingly. Such status data may be stored in a memory of the portable electronic device, e.g. for future reference, as in a data log.

Preferably the information which is provided to the user includes instructions to the user to handle the portable electronic device in a specific manner. Examples will be given below.

The portable first electronic device may be one of the following, without limitation: a mobile phone, a handheld computer, an electronic reader, a tablet computer, a game controller, a pointing device, a photo or a video camera, and a computer peripheral. The portable electronic device will generally comprise an energy source such as a battery to enable the portable electronic device to be operated without connecting the portable electronic device to an electricity network.

The control device may comprise at least one microprocessor and may be configured to run a computer program element (which may be, e.g., part of an application program or a routine carried out by the operating system) containing computer program code that causes the portable electronic device to carry out the aforementioned steps. The computer program element may be stored in a memory of the portable electronic device. The control device may comprise a microprocessor implemented on the same chip as the at least one first sensor, and/or it may comprise one or more other microprocessors of the portable electronic device. The control device need not be implemented in a single piece of hardware; on the contrary, different tasks may be carried out by different hardware elements belonging to the control device. For determining whether the portable electronic device has been handled appropriately during the measurement, the control device may be configured to communicate with a remote server, as will be detailed further below. In particular, the control device may be configured to (autonomously) determine status data indicative of the handling of the portable electronic device, and/or it may be configured to retrieve such status data from a remote server, so as to determine whether the portable electronic device has been handled correctly.

Chemical analytes to which the first sensor is sensitive may include chemical elements and chemical compounds. In particular, the first sensor may be sensitive to one or more of the following: alcohols such as ethanol; ketones such as acetone; aldehydes such as formaldehyde; carbon monoxide; carbon dioxide; ozone; ammonia; methane; benzene and benzene derivatives such as xylenes; thiols, in particular alkylthiols such as methyl mercaptan (methanethiol); nitric oxides (NOx). The analyte will generally be present in a fluid medium, in particular, in a gas, more particularly in air. The first sensor is preferably a semiconductor sensor, in particular, a semiconductor sensor having at least one metal oxide (MOX) layer whose conductivity changes in the presence of at least one chemical analyte. Such sensors will in the following be called MOX sensors. The metal oxide may be, e.g., tin oxide, tungsten oxide, gallium oxide, indium oxide, or zinc oxide. The first sensor may include a heat source integrated within the sensor. The sensor may be manufactured as described, for example, in WO 2012/100362. The sensor may comprise two or more sensor cells that have different sensitivities to selected analytes. The sensor cells may be arranged in a one- or two-dimensional array. The sensor may be implemented on a chip, e.g. a CMOS chip. On the same chip, at least one analog-to-digital converter may be implemented to convert an analog sensor signal into a digital sensor signal. Further digital circuitry may be implemented on the same chip, such as at least one microprocessor for subjecting the digitized signal to at least one preprocessing operation, e.g., baseline correction, scaling, integration, sampling etc..

In a broad sense, the chemical analyte to which the first sensor is sensitive may be water vapor in air, and in this sense, the term "chemical sensor" includes humidity sensors. However, in the following, the term "chemical sensor" will generally be understood to exclude humidity sensors, and a chemical sensor in a more narrow sense will be understood to be a sensor that is sensitive to at least one chemical analyte other than water.

The determination whether the handling of the portable electronic device is correct is carried out with the aid of at least one second sensor that is different from the first sensor. To this end, the control device is configured to determine whether the portable electronic device is handled appropriately based on one or more parameters measured by the second sensor(s), possibly in addition to readings from the first sensor. In particular, the first sensor may be operable to determine a first parameter and the second sensor(s) may be operable to determine at least one second parameter, wherein the second parameter(s) is/are different from the first parameter and indicative of the handling of the portable electronic device. The control device may then be configured to operate the output device to provide information to the user if at least one of the first and second sensors indicates that the handling of the portable electronic device is inappropriate.

In particular, the second sensor(s) includes a sensor selected from the following list: a microphone, a CO₂ sensor, a humidity sensor, an inertial sensor (acceleration sensor), a gyroscope, and a chemical sensor that has a different sensitivity to chemical analytes than the first sensor. In other embodiment that do not belong to the present invention, the second sensor(s) may include: a pressure sensor, a flow sensor, a sensor for determining specific heat of a gas, a sensor for determining density of a gas, a sensor for determining viscosity of a gas, a light sensor (which may be color-sensitive, providing a signal substantially only for one specific wavelength range, or providing a plurality of signals for light in different wavelength regions so as to distinguish between colors), a proximity sensor, a magnetic field sensor, a position sensor, an image sensor, a touch screen, or a joystick.

The second sensor can be a semiconductor sensor. If the first sensor is also a semiconductor sensor, the first and second sensors can be implemented on the same semiconductor chip, i.e., the first and second sensor can form different cells of a common sensor chip. This is, in particular, advantageous if the second sensor is a pressure sensor, a flow sensor, a sensor for determining thermal conductivity of a gas (which, in particular, may be used to determine CO₂ concentration in a gas), a sensor for determining specific heat of a gas, a sensor for determining density of a gas, a sensor for determining viscosity of a gas, a humidity sensor, a temperature sensor, or a chemical sensor that has a different sensitivity to chemical analytes than the first sensor, since these types of sensors can be readily implemented as semiconductor sensors and can readily be integrated on the same chip with a chemical sensor.

In some embodiments, the first sensor may be sensitive to an analyte in air that is exhaled from the user's mouth, and the portable electronic device may be configured to detect this analyte. For instance, the analyte may be ethanol, and the portable electronic device may run an app or a routine of the operating system for determining ethanol levels in the user's breath to obtain a measure of blood alcohol concentration. In other applications, the analyte may be acetone, a thiol, etc. Depending on the second sensor(s), the control device may be configured to do one or more of the following:
(a) If the second sensor is a humidity sensor, the control device may be configured to determine whether humidity measured by the humidity sensor indicates that the user is exhaling air in a predetermined manner. Relative humidity in exhaled air is normally close to 100%. In particular, the control device may be configured to monitor humidity values as a function of time. For instance, if the humidity values indicate a rise in relative humidity to a value above a certain threshold (e.g., in the range of 80%-95%) for a certain time period, this would indicate to the control device that the user is handling the portable electronic device correctly, in particular, that the user is exhaling in the correct direction, with the required intensity and for the required period of time. If no rise in humidity is detected, or if a rise is detected, but is not strong enough (e.g., if the threshold is not reached within a certain time period), or if the rise to too slow, this would indicate that the user is handling the portable electronic device incorrectly, i.e., that the user is not exhaling air in the required manner, and that therefore the first sensor might not deliver meaningful results. More complex criteria may be derived from the humidity values as a function of time. Such criteria may involve numerical integration and/or differentiation and other numerical procedures. If the control device determines that the portable electronic device is handled inappropriately, the control device may operate the output device to provide corresponding information to the user. In particular, the control device may be configured to cause the output device to deliver a message that prompts the user to blow stronger or in a particular direction.
(b) In an embodiment that does not belong to the present invention, the second sensor is a temperature sensor. Then the control device may be configured to determine whether temperature measured by the temperature sensor indicates that the user is exhaling air in a predetermined manner. The temperature of exhaled air is normally close to 37 °C. Similar considerations as for a humidity sensor also apply for a temperature sensor. In particular, the control device may be configured to monitor temperature values as a function of time. For instance, if the temperature values indicate a rise in temperature to a value above a certain threshold (e.g., in the range of 33-35 °C) within a certain time period, this would indicate to the control device that the user is handling the portable electronic device correctly. If no rise in temperature is detected, or if a rise is detected, but is not strong enough (e.g., if the threshold is not reached within a certain time period), or if the rise to too slow, this would indicate that the user is handling the portable electronic device incorrectly. The control device may then operate the output device to provide appropriate information to the user.
(c) In an embodiment that does not belong to the present invention, the second sensor is a flow sensor. Then the control device may be configured to determine whether flow rate measured by the flow sensor indicates that the user is exhaling air in a predetermined manner. Again, similar considerations as for a humidity or temperature sensor also apply for a flow sensor. In particular, the control device may be configured to monitor flow rate values as a function of time. For instance, if the flow rate remains above a certain threshold for a certain time period, this would indicate to the control device that the user is handling the portable electronic device correctly. If no air flow is detected, or if the detected air flow is not strong enough (e.g., if the threshold is not reached), this would indicate that the user is handling the portable electronic device incorrectly. The control device may then operate the output device to provide appropriate information to the user.
(d) If the second sensor is a CO₂ sensor, the control device may be configured to determine whether CO₂ concentration (or, equivalently, partial pressure) measured by the CO₂ sensor indicates that the user is exhaling air in a predetermined manner. CO₂ concentration in exhaled air is normally in the range of about 5-6 vol.-%, whereas CO₂ concentration in ambient air is normally in a range well below 0.1 vol.-%. Again, similar considerations as for a humidity or temperature sensor also apply for a CO₂ sensor. In particular, the control device may be configured to monitor CO₂ concentration values as a function of time. For instance, if the CO₂ concentration values indicate a rise in CO₂ concentration to a value above a certain threshold (e.g., in the range of 2-4 vol.-%) within a certain time period, this would indicate to the control device that the user is handling the portable electronic device correctly. If no rise in CO₂ concentration is detected, or if a rise is detected, but is not strong enough (e.g., if the threshold is not reached within a certain time period), or if the rise to too slow, this would indicate that the user is handling the portable electronic device incorrectly. The control device may then operate the output device to provide appropriate information to the user. CO₂ concentration in a gas may be measured, e.g., by measuring the thermal conductivity of the gas.
(e) If the second sensor is a microphone, the control device may be configured to determine whether sound signals recorded by the microphone indicate that the user is exhaling air in a predetermined manner. Exhalation of air past a microphone will cause a particular noise pattern that may be discriminated from other noise patterns. The exact noise pattern will depend on several factors, including the type and location of the microphone in the housing of the mobile device, the geometry of the channel leading to the microphone, the flow direction of the exhaled air relative to the microphone surface, etc. If a characteristic noise pattern is detected, this would indicate to the control device that the user is handling the portable electronic device correctly. If no such noise pattern is detected, or if a noise pattern is detected, but is below the threshold, this would indicate that the user is handling the portable electronic device incorrectly. The control device may then operate the output device to provide appropriate information to the user.
(f) In an embodiment that does not belong to the present invention, the second sensor is a proximity sensor. Then the control device may be configured to determine whether distance data measured by the proximity sensor indicates that the user holds the portable electronic device in a predetermined distance range from the user's face. If the control device determines that this is not the case, the control device may operate the output device to provide appropriate information to the user. In particular, the control device may be configured to cause the output device to prompt the user to position the portable electronic device closer to the user's mouth.
(g) If the second sensor is an inertial sensor or a gyroscope, the control device may be configured to determine whether measured acceleration and/or orientation of the portable electronic device indicates that the user holds the portable electronic device in a predetermined manner. For instance, if the inertial sensors and/or the gyroscope indicate rapid linear and/or rotational movements of the mobile device, or if the acceleration sensors indicate that the sensor points in an undesired direction (e.g., that the portable electronic device is oriented with its sensor facing downward), this would indicate that the user is handling the portable electronic device incorrectly. The control device may then operate the output device to provide appropriate information to the user. In particular, the control device may be configured to cause the output device to prompt the user to keep the portable electronic device at rest.
(h) In an embodiment that does not belong to the present invention, the second sensor is an image sensor (in particular, a camera). Then the control device may be configured to determine whether image data recorded by the image sensor indicates that the user is holding the portable electronic device in a predetermined manner. In particular, the control device may be configured to carry out a pattern recognition algorithm to determine whether the image sensor is recording a human face. Such face recognition algorithms are well known in the art. If no human face is recorded, this would indicate that the user is handling the portable electronic device incorrectly. The control device may then operate the output device to provide appropriate information to the user. In particular, the control device may be configured to cause the output device to prompt the user to turn the portable electronic device so as to face the user.
(j) If the second sensor is a chemical sensor that has a different sensitivity to chemical analytes than the first sensor, the control device may be configured to employ the second sensor to determine the presence and/or concentration of at least one trace metabolite in the exhaled air. Such trace metabolites include ammonia, acetone, methanol, ethanol, 2-methyl-1,3-butadiene (isoprene), hydrogen, methane, hydrogen cyanide, and hydrogen sulfide. Such trace metabolites are released by the body into the air when air is exhaled. At least for some of these metabolites, the concentration of the metabolite in the exhaled air is largely independent of the concentration of the respective metabolite in the blood stream. However, the concentration in the exhaled air can strongly depend on whether the air is exhaled through the mouth or through the nose. For instance, air exhaled through the mouth contains an ammonia concentration that is up to ten times higher than for air exhaled through the nose. By measuring the presence and/or concentration of trace metabolites in the exhaled air, one can therefore determine whether the user exhales air in a predetermined manner, in particular, whether the user exhales air through the mouth or through the nose. The control device may then operate the output device to provide appropriate information to the user. If, for instance, the measurement indicates that air is exhaled through the nose, the control device may be configured to cause the output device to prompt the user to exhale air through the mouth instead. The trace metabolite should of course be different from the chemical analyte that is intended to be primarily detected by the portable electronic device. For instance, if the portable electronic device is configured to detect ethanol in air that is exhaled from the user's mouth, the trace metabolite should not be ethanol.

In other embodiments that do not belong to the present invention, the first sensor may be sensitive to an analyte in a gas received from a container, e.g., from a bottle or a drinking vessel, or in a gas received from an object, e.g., from a fruit or a wet sponge, and the portable electronic device may be configured to detect this analyte. For instance, the portable electronic device may be configured to detect whether the container is filled with an alcoholic drink (by detecting ethanol in the vapor emanating from the vessel), with coffee (by detecting analytes that are characteristic for coffee scent), with sparkling drinks (by detecting CO₂) etc., to detect whether the object is a particular fruit, to detect whether the object emits certain gases, e.g, noxious gases, etc. Depending on the second sensor(s), the control device may be configured to do one or more of the following:
(a) If the second sensor is a humidity sensor, the control device may be configured to determine whether humidity measured by the humidity sensor indicates that the user has positioned the portable electronic device in proximity to an opening of a container filled with a (possibly hot) aqueous liquid or in proximity to a wet object. Relative humidity in air emanating from a vessel filled with an aqueous liquid or from a wet object will normally be much higher than the relative humidity of the surrounding air. This is particularly true if the container is filled with a hot liquid like hot coffee or tea, or if the wet object is hot. In particular, the control device may be configured to monitor humidity values as a function of time. For instance, if the humidity values indicate a rise in relative humidity to a value above a certain threshold (e.g., in the range of 70%-90%) within a certain time period, this would indicate to the control device that the user is handling the portable electronic device correctly, in particular, that the user has placed the portable electronic device close to the opening of a container containing a (possibly hot) aqueous liquid or close to a (possibly hot) wet object. If no rise in humidity is detected, or if a rise is detected, but is not strong enough (e.g., if the threshold is not reached within a certain time period), or if the rise to too slow, this could indicate that the user is handling the portable electronic device incorrectly, e.g., that the user has not placed the device sufficiently close to the opening, or that the container is empty, or that the object is dry, and that therefore the first sensor might not deliver meaningful results. If the control device determines that the portable electronic device is handled inappropriately, the control device may operate the output device to provide corresponding information to the user. In particular, the control device may be configured to cause the output device to deliver a message that prompts the user to check the position of the portable electronic device and to ensure that the container is indeed filled with an aqueous liquid.
(b) If the second sensor is a temperature sensor, the control device may be configured to determine whether temperature measured by the temperature sensor indicates that the user has positioned the portable electronic device in proximity to a container containing a hot fluid or in proximity to a hot object. Gases emanating from a container containing a hot fluid or from a hot object will generally be much warmer than the ambient air. Similar considerations as for a humidity sensor also apply for a temperature sensor. In particular, the control device may be configured to monitor temperature values as a function of time. For instance, if the temperature values indicate a rise in temperature to a value above a certain threshold (e.g., in the range of 45-60 °C) within a certain time period, this would indicate to the control device that the user is handling the portable electronic device correctly, and that the fluid in the container or the object is hot. If no rise in temperature is detected, or if a rise is detected, but is not strong enough (e.g., if the threshold is not reached within a certain time period), or if the rise to too slow, this would indicate that the user is handling the portable electronic device incorrectly, or that no hot fluid is present in the container, or that the object is not hot. The control device may then operate the output device to provide appropriate information to the user.
(c) If the second sensor is a chemical sensor that is sensitive to a further chemical analyte, the control device may be configured to determine whether a concentration of the further chemical analyte measured by the second sensor indicates that the user has positioned the portable electronic device in proximity to an object or container emitting said further chemical analyte. For instance, if the second sensor is a CO₂ sensor, the control device may be configured to determine whether CO₂ concentration (or, equivalently, partial pressure) measured by the CO₂ sensor indicates that the user has positioned the portable electronic device in proximity to a container containing a carbonated liquid. CO₂ concentration in ambient air is normally in a range well below 0.1 vol.-%. Gases emanating from a container containing a carbonated liquid will generally have a higher CO₂ content. In another embodiment, the second sensor may be an ethanol sensor to detect whether the portable electronic device is positioned close to the opening of a container containing an alcoholic drink. Similar considerations as for a humidity or temperature sensor also apply for a sensor that is sensitive to a further chemical analyte. In particular, the control device may be configured to monitor concentration values for the further chemical analyte as a function of time. For instance, if the concentration values of the further chemical analyte indicate a rise in said concentration to a value above a certain threshold within a certain time period, this would indicate to the control device that the user is handling the portable electronic device correctly. If no rise in the concentration of the further chemical analyte is detected, or if a rise is detected, but is not strong enough (e.g., if the threshold is not reached within a certain time period), or if the rise to too slow, this would indicate that the user is handling the portable electronic device incorrectly. For instance, if the further chemical analyte is CO₂, a missing or very slow rise may indicate that the user has not placed the portable electronic device properly near the opening of the container, or that no carbonated liquid is present in the container. The control device may then operate the output device to provide appropriate information to the user.
(d) If the second sensor is a microphone, the control device may be configured to determine whether sound signals recorded by the microphone indicate that the user has positioned the portable electronic device in proximity to a container containing a sparkling liquid or to an object that emits sounds. A sparkling liquid will cause a particular noise pattern that can be discriminated from other noise patterns. If a characteristic noise pattern is detected and is above a certain intensity threshold, this would indicate to the control device that the user is handling the portable electronic device correctly and that the container contains a sparkling liquid. If no such noise pattern is detected, or if a noise pattern is detected, but is below the threshold, this would indicate that the user is handling the portable electronic device incorrectly, or that the no sparkling liquid is present. The control device may then operate the output device to provide appropriate information to the user.
(e) If the second sensor is a proximity sensor, the control device may be configured to determine whether distance data measured by the proximity sensor indicates that the user has positioned the portable electronic device in a predetermined distance range from the container or object. If the control device determines that this is not the case, the control device may operate the output device to provide appropriate information to the user. In particular, the control device may be configured to cause the output device to prompt the user to position the portable electronic device closer to the container or object.
(f) If the second sensor is an inertial sensor or a gyroscope, the control device may be configured to determine whether measured acceleration and/or orientation of the portable electronic device indicates that the user has positioned the portable electronic device in a predetermined manner relative to the container or object. For instance, if the acceleration sensors and/or the gyroscope indicate rapid linear and/or rotational movements of the mobile device, or if the inertial sensors indicate that the portable electronic device points in an undesired direction (e.g., that the portable electronic device is oriented with the sensor facing upward), this would indicate that the user is handling the portable electronic device incorrectly. The control device may then operate the output device to provide appropriate information to the user. In particular, the control device may be configured to cause the output device to prompt the user to keep the portable electronic device at rest.
(g) If the second sensor is an image sensor (in particular, a camera), the control device may be configured to determine whether image data recorded by the image sensor indicates that the user has positioned the portable electronic device in a predetermined manner relative to the container or object. In particular, the control device may be configured to carry out a pattern recognition algorithm to determine whether the image sensor is recording the image of a vessel or of a certain predetermined object (e.g., a fruit or a sponge). If no image of a vessel or of an object of the expected type is recorded, this could indicate that the user is handling the portable electronic device incorrectly. The control device may then operate the output device to provide appropriate information to the user. In particular, the control device may be configured to cause the output device to prompt the user to position the portable electronic device close to a vessel or an expected type of object.

In still other embodiments that do not belong to the present invention, the first sensor may be sensitive to an analyte in an environmental gas, e.g., in ambient air, and the portable electronic device may be configured to detect this analyte. For instance, the portable electronic device may be configured to detect CO or O₃ in ambient air. Depending on the second sensor(s), the control device may be configured to do one or more of the following:
(a) If the second sensor is a proximity sensor, the control device may be configured to determine whether distance data measured by the proximity sensor indicates that the user has positioned the portable electronic device in an enclosed space. For instance, if the user has placed the portable electronic device in a closed protective cover, the device will not be able to receive enough ambient air to deliver meaningful results. The proximity sensor will detect that the device is covered with material, and the control device may operate the output device to provide appropriate information to the user. In particular, the control device may be configured to cause the output device to prompt the user to position the portable electronic device outside an enclosed space, sufficiently far removed from structures that would impede ambient air reaching the device.
(b) If the second sensor is a light sensor, the control device may be configured to determine whether light intensity measured by the light sensor indicates that the user has positioned the portable electronic device in an enclosed space. For instance, if the user has placed the portable electronic device in a closed protective cover, the device will not be able to receive enough ambient air to deliver meaningful results. The light sensor will detect that no ambient light reaches the device, and the control device may operate the output device to provide appropriate information to the user. In particular, the control device may be configured to cause the output device to prompt the user to position the portable electronic device outside an enclosed space, where it is accessible to ambient light.
(c) If the second sensor is an acceleration sensor or a gyroscope, the control device may be configured to determine whether measured acceleration and/or orientation of the portable electronic device indicates that the user is moving the portable electronic device. Rapid movements of the device can accelerate diffusion of the ambient air to the sensor surface. For instance, if the user is keeping the device stationary, the control device may operate the output device to provide appropriate information to the user. In particular, the control device may be configured to cause the output device to prompt the user to rapidly move ("shake") the device to accelerate air exchange at the sensor surface.
(d) If the second sensor is a flow sensor, the control device may be configured to determine whether the user is moving the portable electronic device, as discussed above.
(e) The readings of an acceleration sensor and a flow sensor may also be combined to differentiate between two scenarios. For instance, if the second sensors include a flow sensor and an acceleration sensor, the control device may be configured to determine whether a flow detected by the flow sensor corresponds to a movement of the electronic device or whether the flow is induced by convection.
(f) If the second sensor is a temperature sensor, the control device may be configured to determine whether temperature is stable.
(g) If the second sensor is a humidity sensor, determining whether humidity is stable.

If temperature and/or humidity are not stable, this may indicate that the environment is changing, e.g., by a user just taking the electronic device out of a closed environment like a pocket. In this context, humidity and/or temperature data may be further correlated with data from an acceleration sensor or a gyroscope so as to obtain an improved indication whether the environment of the portable electronic device is stable.

As has already become apparent, in all these embodiments, it is of course possible to provide not only a single second sensor, but two or more second sensors, each of the second sensors measuring a certain parameter, and to combine the parameters measured by the different second sensors when determining whether the portable electronic device is handled correctly. For instance, the control device might determine that the portable electronic device is handled correctly only if the readings of all the second sensors indicate that this is the case. In other embodiments the control device might derive or obtain one or more indicators for the extent to which the portable electronic device is handled correctly, based on the readings of the second sensors, each of the second sensors being assigned a certain weight in the calculation of the indicator. If the indicator exceeds a threshold, the control device may decide that the device is handled correctly.

The handling conditions determined by the present method and/or the data from the one or more second sensors may not only be used for determining whether the portable electronic device has been handled correctly, but may instead or in addition be employed for data analysis of the data obtained from the first sensor. For instance, if one of the second sensors is a humidity sensor, the reading of that sensor may not only be used to determine whether the portable electronic device has been operated in the correct atmosphere, but it may also be used to correct the reading of the first sensor for a known humidity dependence of the signal of the first sensor. Many other such possibilities exist. In this manner, the quality of the results obtained by the measurement can be improved. In addition, from the knowledge of the measurement conditions, an estimator for the quality of the results may be derived. The data analysis of the data from the first sensor may be carried out in the portable electronic device, on a remote server, or it may be split between the portable electronic device and a remote server.

The portable electronic device may further comprise a communication module for data exchange with a remote server (or the "cloud") through a digital communication channel. The communication module may be a wireless communication module for communication through a wireless communication channel. Often, the portable electronic device will comprise a plurality of such communication modules. Such modules may include at least one module for connecting the portable electronic device to a wireless telephony network, e.g., a GPRS module, a UMTS module, or an LTE module, or it may include a Wi-Fi module, a Bluetooth module, a near-field communication module etc. or any other module that allows the exchange of data, in particular, the exchange of data via a data network, including the Internet. The wireless communication module may comprise an antenna. The control device may then be configured to send sensor data to a remote server via the wireless communication module and to receive, in response, status data indicative of the handling of the portable electronic device from said remote server via the wireless communication module, based on the sensor data sent to the remote server. The sensor data may comprise data from the first and/or second sensors. In other words, all or part of the algorithms for determining whether the portable electronic device is handled appropriately may be carried out on a remote server ("cloud computing"). However, it is also possible that all algorithms are carried out entirely locally in the portable electronic device.

The output device may be, e.g., a display, a loudspeaker, a vibrator, a flash, a beamer, an LED, or a laser. More than one output device may be employed. For instance, if the output device is a display, the control device may drive the display to provide information to the user by showing a text message and/or a graphical symbol that prompts the user to operate the portable electronic device is the desired manner. If the output device is a loudspeaker, the control device may drive the loudspeaker to provide information to the user by outputting a spoken message or a certain sound that prompts the user to operate the portable electronic device is the desired manner.

The method of claim 5 is a method for operating a portable electronic device comprising at least one first sensor, the first sensor being sensitive to at least one chemical analyte. The method comprises:
carrying out a measurement with the first sensor;
determining whether the portable electronic device has been handled appropriately during the measurement; and
if the portable electronic device has not been handled appropriately, providing information to a user of the portable electronic device.

As discussed above, the information may include instructions to the user to handle the portable electronic device in a specific manner.

As discussed above, the portable electronic device may comprise at least one second sensor. The determination whether the portable electronic device has been handled appropriately during the measurement may then be based on one or more parameters measured by the second sensor.

The method may be entirely computer-implemented. As described before, the steps of the method of the present invention may be carried out by a control device of the portable electronic device, and the control device may comprise a microprocessor. As discussed above, the method may involve sending sensor data to a remote server via a wireless communication module and to receive, in response, status data indicative of the operating condition of the first sensor from said remote server via the wireless communication module, based on the sensor data sent to the remote server.

All considerations that are discussed in this document in conjunction with the portable electronic device of the present invention are equally applicable to the method of the present invention.

In another aspect, the present invention provides, in claim 8, a computer program element comprising computer program code that, when executed in a control device of a portable electronic device comprising at least one first sensor for determining a first parameter, carries out the method of the present invention.

The present invention further provides, in claim 9, a method of operating a determination unit in communication with a portable electronic device, the method comprising:
receiving sensor data from a portable electronic device comprising at least one first sensor, the first sensor being sensitive to at least one chemical analyte;
based on the sensor data, deriving status data indicative of the handling of the portable electronic device; and
sending said status data to the portable electronic device.

The sensor data may comprise data from at least one first sensor and/or at least one second sensor, as detailed above. The present invention further provides a determination unit configured for carrying out said method and a computer program element comprising computer code that, when executed in a processor of a determination unit, carries out said method.

Further disclosed is a portable electronic device, comprising:
a control device;
at least one first sensor, the first sensor being sensitive to at least one chemical analyte, the first sensor being coupled to the control device;
at least one second sensor coupled to the control device, the at least one second sensor being selected from the following list: a microphone, a pressure sensor, a flow sensor, a sensor for determining thermal conductivity of a gas (which, in particular, may be used to determine CO₂ concentration in a gas), a sensor for determining specific heat of a gas, a sensor for determining density of a gas, a sensor for determining viscosity of a gas, a humidity sensor, a temperature sensor, a light sensor (which may be color-sensitive, providing a signal substantially only for one specific wavelength range, or providing a plurality of signals for light in different wavelength regions so as to distinguish between colors), a proximity sensor, an inertial sensor (acceleration sensor), a gyroscope, a magnetic field sensor, a position sensor, an image sensor, a touch screen, a joystick, and a chemical sensor that has a different sensitivity to chemical analytes than the first sensor;
wherein the control device is configured to carry out the following steps:
   carrying out a measurement with the first sensor and the at least one second sensor;
   analyzing data from the first sensor taking into account one or more parameters measured by the at least one second sensor.

In other words, data obtained from a chemical sensor and from one or more additional sensors, which measure other parameters, in particular, physical parameters, are correlated. This is advantageous independent of whether or not a determination is carried out whether a user has handled the portable electronic device correctly.

By correlating data obtained from a chemical sensor and from one or more additional sensors, the quality of the results obtained by the chemical sensor can be improved. The additional sensors may be used to determine the measurement conditions to which the chemical sensor is exposed so as to be able to take the measurement conditions into account in data analysis; and/or the additional sensors may be used to directly correct a reading of the chemical sensor, e.g., for dilution effects. In addition, from the knowledge of the measurement conditions as measured by the second sensor(s), an estimator for the quality of the results may be derived.

The data analysis may be carried out entirely in the portable electronic device, on a remote server, or it may be split between the portable electronic device and a remote server. In other words, data analysis may comprise sending data derived from measurements with the first and second sensors to a remote server and receiving result data from the remote server, and the result data may or may not be further analyzed by the control device of the portable electronic device. Preferably the control device is configured to operate an output device of the portable electronic device to present the result data or information derived therefrom to a user of the portable electronic device.

Further disclosed is a corresponding method for operating a portable electronic device. The portable electronic device comprises at least one first sensor, the first sensor being sensitive to at least one chemical analyte, and at least one second sensor, the at least one second sensor being selected from the following list: a microphone, a pressure sensor, a flow sensor, a sensor for determining thermal conductivity of a gas, a sensor for determining specific heat of a gas, a sensor for determining density of a gas, a sensor for determining viscosity of a gas, a humidity sensor, a temperature sensor, a light sensor. The method comprises:
carrying out a measurement with the first sensor and the at least one second sensor; and
analyzing data from the first sensor taking into account one or more parameters measured by the at least one second sensor.

Further disclosed is a computer program element comprising computer code that, when executed in a processor of a determination unit, carries out said method.

All considerations that are discussed in this document in conjunction with the portable electronic devices and the methods of the present invention are equally applicable to the computer program elements of the present invention.

The computer program elements may be provided in any suitable form, including source code or object code. In particular, they may be stored on a computer-readable medium or embodied in a data stream. The data stream may be accessible through a network such as the Internet.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: is a perspective view of a portable electronic device;
- Fig. 2: is a highly schematic view illustrating the various components in a portable electronic device;
- Fig. 3: is a flow diagram illustrating a method of operating the portable electronic device;
- Fig. 4: is a diagram schematically illustrating the readings of different sensors in a breath analysis measurement; and
- Figs. 5-7: are schematic illustrations of exemplary use cases.

### DESCRIPTION OF PREFERRED EMBODIMENTS

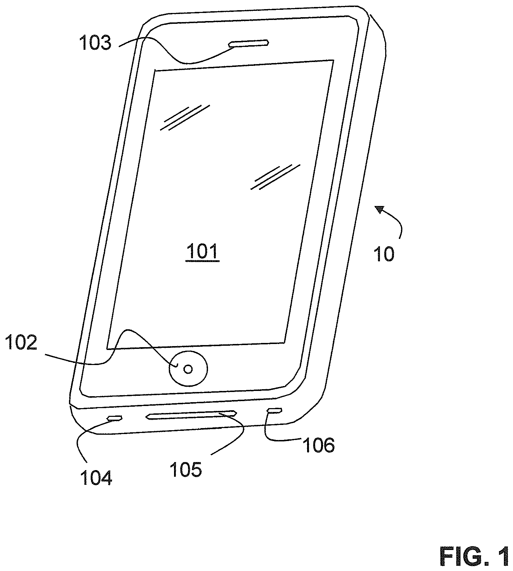

Figure 1 illustrates a portable electronic device 1 in the form of a mobile phone. The housing 10 of the mobile phone includes a front side with a touchscreen display 101, a switch button 102 and an opening 103 for a first loudspeaker. In a lower sidewall region of the housing 10, further openings 104, 105 and 106 are provided. Behind these openings, components such as a microphone, further loudspeakers and connectors are disposed. In addition, behind any of these openings sensors such as a humidity sensor, a temperature sensor and one or more sensors for detecting at least one chemical analyte (i.e., one or more chemical sensors) may be arranged. Each chemical sensor can comprise one or more sensor cells, each sensor cell exhibiting a different sensitivity to selected analytes.

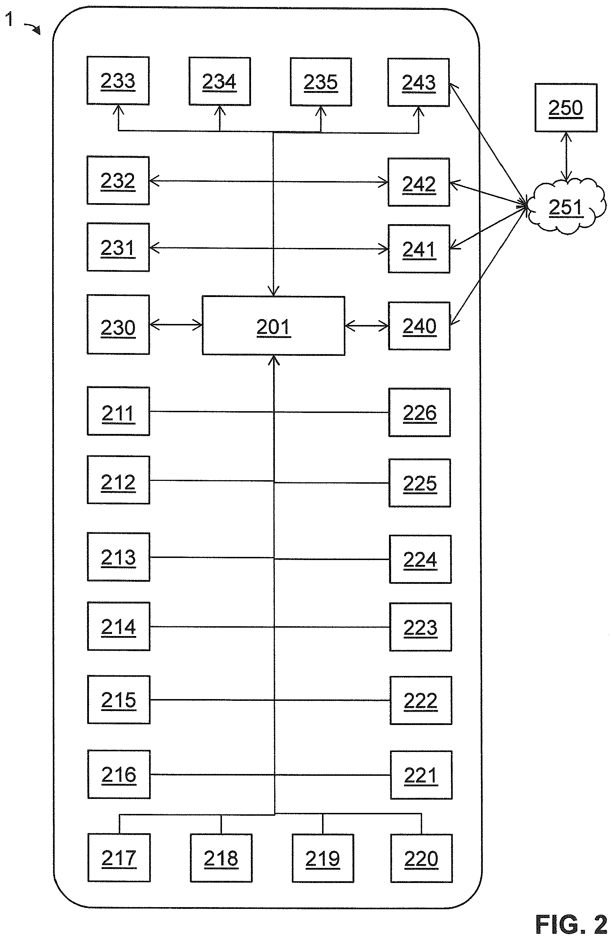

In Figure 2, the various components that may be present in a portable electronic device are schematically illustrated. A control device 201, which will generally comprise a microprocessor and a memory, interacts with a number of input/output devices and other modules, as described in the following. Input devices may be present, e.g., as follows:
- a microphone 211;
- a front camera 212;
- a rear camera 213;
- three orthogonal inertial sensors (linear acceleration sensors) 214;
- three orthogonal gyroscopes (rotation sensors) 215;
- a magnetometer 216;
- a GPS module 217;
- a proximity sensor 218;
- a light intensity sensor 219;
- a pressure sensor 220;
- a temperature sensor 221;
- a humidity sensor 222;
- a flow sensor 223;
- a sensor 224 for measuring thermal conductivity of the surrounding gas; such sensor may be used for measuring CO₂ content in a gas;
- a sensor 225 for measuring specific heat of the surrounding gas; and
- a chemical sensor 226.

Furthermore, a combined input/output device in the form of the touchscreen display 230 may be present.

Output devices may be present as follows:
- a front loudspeaker 231 for telephony;
- at least one bottom loudspeaker 232 for hands-free operation;
- a vibrator 233;
- a flash 234;
- a status LED 235;
- a beamer 236.

Furthermore, the portable electronic device may comprise one or more wireless communication modules for exchanging data with a remote server 250 through a wireless data connection 251, such as a GPRS module 240, a UMTS module 241, a WLAN module 242 and a Bluetooth module 243. Of course, more such modules may be provided.

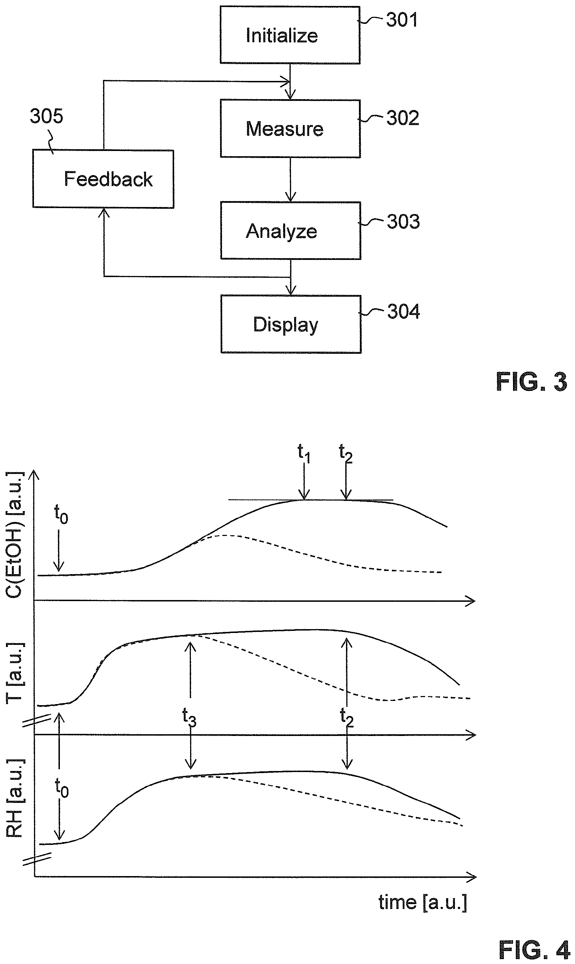

In the memory of the control device 201, an application program (app) and/or a routine of the operating system (a "chemical engine") may be stored for carrying out measurements with the chemical sensor 226. Figure 3 schematically illustrates a flow diagram for such a measurement.

In a first step 301, the portable electronic device is initialized. To this end, parameters needed for the measurements are downloaded from the remote server (from the "cloud"). The chemical sensor 226 is then heated up to its operational temperature. When the sensor is ready to take measurements, the control device 201 will cause the display 230 or the loudspeaker 232 to output a corresponding message to the user, instructing the user to begin with the measurement.

In a second step 302, a measurement cycle this carried out. To this end, a plurality of sensors is operated to obtain readings from these sensors. In particular, conductivity values are measured for the chemical sensor 226, and readings are taken from other sensors among the sensors 211-225, which will indicate whether or not the user handles the portable electronic device in an appropriate manner. All obtained sensor data are recorded by the control device.

In a third step 303, the obtained sensor data are analyzed by the control device. Analysis may take place entirely within the control device using algorithms of the app itself, or the control device may send the raw sensor data or pre-processed sensor data to a remote server 250 (to the "cloud") with the aid of the wireless communication module 240. In this case, the remote server 250 would carry out at least part of the analysis and send data that represent the results of the analysis back to the portable electronic device 1. The portable electronic device would then receive these data through the wireless communication module 240. The analysis involves a determination whether the portable electronic device has been handled correctly during the measurement cycle. To this end, the data of one or more of the sensors 211-225 are analyzed. Furthermore, the analysis involves a derivation of an output parameter from the conductivity values of the chemical sensor 226.

If the analysis of the data of the sensors 211-225 shows that the portable electronic device has been handled appropriately, the control device will cause at least one of the output devices 231-236 to output the results of the measurement, e.g. it will cause the display to show a text message or a graphical representation containing the results (step 304).

If, on the other hand, the analysis of the data of the sensors 211-225 shows that the portable electronic device has not been handled appropriately, the control device will cause at least one of the output devices 231-236 to output a corresponding feedback message to the user (step 305). The message will contain instructions to the user on how the user should actually handle the portable electronic device. Thereafter, a new measurement cycle is started, either immediately or after the user has acknowledged that the feedback message has been noticed.

For instance, if a measurement is carried out for determining breath alcohol content, the analysis in step 303 involves a determination whether the user has exhaled with sufficient intensity and for a sufficient amount of time towards the sensor surface. To this end, data of the temperature sensor 221 and the humidity sensor 222 may be employed.

Figure 4 shows a diagram schematically illustrating the response of the temperature sensor 221 (apparent temperature T in arbitrary units), the humidity sensor 222 (apparent relative humidity RH in arbitrary units) and a chemical sensor 226 that is sensitive to ethanol (apparent ethanol concentration c(EtOH) in arbitrary units) during an exhalation of the user. Two scenarios are illustrated. In the first scenario, the user exhales towards the portable electronic device in the correct direction, with sufficient intensity and for a sufficient amount of time (solid lines in Figure 4). In the second scenario, the user exhales towards the portable electronic device in the correct direction, with sufficient intensity, but for an insufficient amount of time (broken lines in Figure 4).

In both scenarios, exhalation starts at a point in time to. Warm and humid exhaled air from the user's mouth and lungs reaches the surface of the three sensors 221, 222 and 226. The three sensors 221, 222 and 226 will have different response times to this stimulus. In the present example, the response of the temperature sensor 221 is the fastest, resulting in relatively rapid rise of apparent temperature T with time. The response of the humidity sensor 222 is slightly slower, resulting in a slightly slower rise of apparent relative humidity RH with time. The chemical sensor 226 exhibits the slowest response, resulting in a delayed rise of apparent ethanol concentration c(EtOH) with time.

In the first scenario, at a point t₁, the reading of the chemical sensor 226 has reached a plateau and has become essentially stationary. At this point in time, the reading of the chemical sensor 226 more or less accurately reflects the actual ethanol concentration in the exhaled air. At a point t₂, the user stops exhaling, and the readings of all three sensors start to fall again.

In the second scenario, the user stops exhaling already at a point t₃, which is before the reading of the chemical sensor 226 has reached the plateau. The reading of the chemical sensor 226 will therefore never reflect the actual ethanol concentration in the exhaled air.

The control device can detect whether the user has exhaled with sufficient intensity and for a sufficient amount of time by taking the following data into account: the points in time at which the readings of each of the temperature sensor and the humidity sensor have started to rise; the points in time at which the readings of each of these sensors have essentially reached a plateau (or, equivalently any other suitable measure of rise time of the corresponding sensor); the points in time at which the readings of these sensors have started to fall again; the plateau value of the apparent temperature T; and the plateau value of the apparent relative humidity. For instance, a rapid rise to a sufficiently high plateau of both temperature and relative humidity will indicate that the user has exhaled towards the portable electronic device in the right direction and with sufficient intensity, and a sufficiently long time span between the points in time when the plateau values of temperature and relative humidity have been reached and the points in time at which temperature and relative humidity start to fall again will indicate that the user has exhaled towards the portable electronic device for a sufficient length of time. Of course, it is possible to derive more complex criteria from the readings of these sensors, in particular, criteria that correlate the readings of the different sensors.

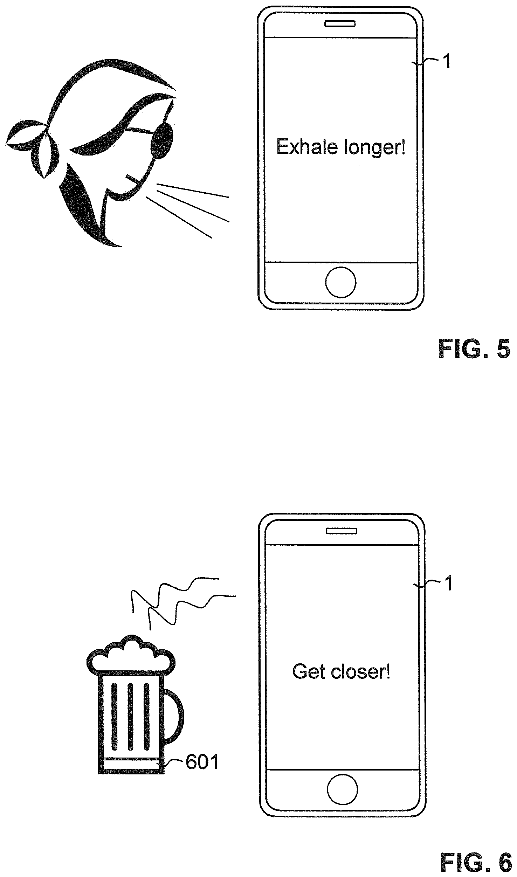

In the present example, in the second scenario the control device will detect that the readings of the temperature sensor and the humidity sensor start to fall again already from the point t₃. The control device will realize that the time span between the start of the rise of the temperature and humidity and the point t₃ has been too short for the chemical sensor to reach its stationary state (its plateau), and will therefore determine that the portable electronic device has not been handled correctly. The control device will therefore cause one or more of the output devices to output a message informing the user that he should exhale for a longer amount of time (Figure 5).

Instead of a temperature sensor and a humidity sensor or in addition to these, other types of sensors may be employed for determining whether the portable electronic device has been handled correctly. For instance, if the portable electronic device comprises a flow sensor, air flow could be monitored as a function of time to determine whether the user has exhaled for a sufficient amount of time. Inertial sensors and gyroscopes may be used to determine whether the portable electronic device is kept at rest, etc.

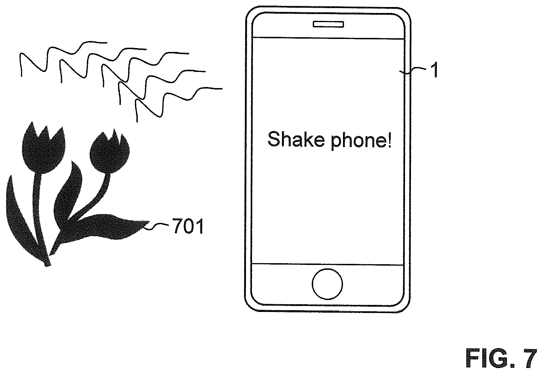

Figures 6 and 7 illustrate two further use cases, which are not part of the present invention.

In Figure 6, the portable electronic device 1 is used to detect what kind of liquid is contained in a drinking vessel 601. To this end, the portable electronic device comprises at least one chemical sensor which is sensitive to at least one analyte that is typically present in the liquids of interest. In order to obtain meaningful results, the portable electronic device 1 should be positioned in such a manner that the opening in the housing 10 behind which the chemical sensor is arranged is placed just above the vessel 601. In order to determine whether this is the case, various other sensors may be employed, including a proximity sensor to determine whether the portable electronic device is sufficiently close to the vessel 601, a humidity sensor to determine whether the portable electronic device receives water vapor emanating from the vessel, a front camera of the portable electronic device to determine whether the portable electronic device faces a drinking vessel, or inertial sensors and gyroscopes to determine whether the portable electronic device is kept at rest.

In Figure 7, the portable electronic device is used to detect the scent of a flower 701. To this end, the portable electronic device comprises at least one chemical sensor which is sensitive to at least one analyte that is characteristic of the typical scents of flowers. In order to obtain meaningful results, the portable electronic device 1 should be moved back and forth ("shaken") close to the flower to ensure that a sufficient amount of air reaches the chemical sensor within a reasonable time span. In order to determine whether this is the case, inertial sensors and gyroscopes in the portable electronic device may be used.

## Claims

1. A portable electronic device (1) configured to detect at least one chemical analyte in air that is exhaled from a user's mouth, comprising:
a control device (201);
at least one first sensor (226), the first sensor (226) being sensitive to at least one chemical analyte, the first sensor (226) being coupled to the control device (201);
at least one second sensor (211-225) coupled to the control device (201); and
at least one output device (230-236) coupled to the control device;
wherein the control device is configured to carry out the following steps:
carrying out a measurement with the first sensor (226);
determining whether the portable electronic device (1) has been handled appropriately during the measurement; and
if the portable electronic device (1) has not been handled appropriately, operating the output device (230-236) to present information to a user of the portable electronic device (1),
**characterized in that**
the at least one second sensor (211-225) is a humidity sensor, a CO₂ sensor, a microphone, an acceleration sensor, a gyroscope, or a second chemical sensor that has a different sensitivity to chemical analytes than the first sensor, and **in that**
the determination whether the portable electronic device (1) has been handled appropriately during the measurement is based on at least one of the following:
(a) if the second sensor is a humidity sensor, determining whether humidity measured by the humidity sensor exceeds a threshold for a time period so as to indicate that the user is exhaling air in a predetermined manner;
(b) if the second sensor is a CO₂ sensor, determining whether CO₂ concentration measured by the CO₂ sensor rises above a threshold within a time period so as to indicate that the user is exhaling air in a predetermined manner;
(c) if the second sensor is a microphone, determining whether sound signals recorded by the microphone correspond to a characteristic noise pattern so as to indicate that the user is exhaling air in a predetermined manner;
(d) if the second sensor is an acceleration sensor or a gyroscope, determining whether measured acceleration or orientation of the portable electronic device indicates that the portable electronic device is being subjected to rapid linear or rotational movements, or that the portable electronic device is pointing in an undesired direction, so as to indicate whether the user is holding the portable electronic device in a predetermined manner;
(e) if the second sensor is a chemical sensor that has a different sensitivity to chemical analytes than the first sensor, determining whether the presence or concentration of at least one trace metabolite in the exhaled air indicates whether the user is exhaling air through his mouth or through his nose.

2. The portable electronic device according to claim 1, wherein the information to the user includes instructions to handle the portable electronic device (1) in a specific manner.

3. The portable electronic device according to any one of the preceding claims, wherein the portable electronic device (1) comprises at least two second sensors, each of the second sensors being operable to measure a different parameter, and wherein the control device is configured to combine at least two parameters measured by different second sensors when determining whether the portable electronic device (1) has been handled correctly.

4. The portable electronic device according to any one of the preceding claims, wherein the portable electronic device comprises at least one communication module (240, 241, 242), and wherein the control device (201) is configured to send sensor data to a remote server (250) via the at least one communication module and to receive, in response, status data indicative of the handling of the portable electronic device from said remote server via the at least one communication module.

5. A method for operating a portable electronic device (1) comprising at least one first sensor (226), the first sensor (226) being sensitive to at least one chemical analyte, and at least one second sensor, the method comprising:
carrying out a measurement with the first sensor (226);
determining whether the portable electronic device (1) has been handled appropriately during the measurement; and
if the portable electronic device (1) has not been handled appropriately, providing information to a user of the portable electronic device (1),
**characterized in that**
the at least one second sensor is a humidity sensor, a CO₂ sensor, a microphone, an acceleration sensor, a gyroscope, or a second chemical sensor that has a different sensitivity to chemical analytes than the first sensor, and **in that**
the determination whether the portable electronic device (1) has been handled appropriately during the measurement is based on at least one of the following:
(a) if the second sensor is a humidity sensor, determining whether humidity measured by the humidity sensor exceeds a threshold for a time period so as to indicate that the user is exhaling air in a predetermined manner;
(b) if the second sensor is a CO₂ sensor, determining whether CO₂ concentration measured by the CO₂ sensor rises above a threshold within a time period so as to indicate that the user is exhaling air in a predetermined manner;
(c) if the second sensor is a microphone, determining whether sound signals recorded by the microphone correspond to a characteristic noise pattern so as to indicate that the user is exhaling air in a predetermined manner;
(d) if the second sensor is an acceleration sensor or a gyroscope, determining whether measured acceleration or orientation of the portable electronic device indicates that the portable electronic device is subjected to rapid linear or rotational movements, or that the portable electronic device points in an undesired direction, so as to indicate whether the user is holding the portable electronic device in a predetermined manner;
(e) if the second sensor is a chemical sensor that has a different sensitivity to chemical analytes than the first sensor, determining whether the presence or concentration of at least one trace metabolite in the exhaled air indicates whether the user is exhaling air through his mouth or through his nose.

6. The method according to claim 5, wherein the information includes instructions to the user to handle the portable electronic device (1) in a specific manner.

7. The method according to claim 5 or 6, comprising sending sensor data to a remote server (250) and receiving, in response, status data indicative of the handling of the portable electronic device from said remote server.

8. A computer program element comprising computer code that, when executed in a control device (201) of a portable electronic device (1) comprising at least one first sensor (226), the first sensor (226) being sensitive to at least one chemical analyte, carries out the method of any one of claims 5-7.

9. A method of operating a determination unit at a remote server (250), the determination unit being in communication with a portable electronic device according to claim 4, the method comprising:
receiving sensor data from the portable electronic device (1) at the remote server (250), the sensor data comprising data derived from measurements with the first and second sensors;
deriving status data indicative of the handling of the portable electronic device (1); and
sending said status data to the portable electronic device (1),
wherein the status data are derived based on at least one of the following:
(a) if the second sensor is a humidity sensor, determining whether humidity measured by the humidity sensor exceeds a threshold for a time period so as to indicate that the user is exhaling air in a predetermined manner;
(b) if the second sensor is a CO₂ sensor, determining whether CO₂ concentration measured by the CO₂ sensor rises above a threshold within a time period so as to indicate that the user is exhaling air in a predetermined manner;
(c) if the second sensor is a microphone, determining whether sound signals recorded by the microphone correspond to a characteristic noise pattern so as to indicate that the user is exhaling air in a predetermined manner;
(d) if the second sensor is an acceleration sensor or a gyroscope, determining whether measured acceleration or orientation of the portable electronic device indicates that the portable electronic device is subjected to rapid linear or rotational movements, or that the portable electronic device points in an undesired direction, so as to indicate whether the user is holding the portable electronic device in a predetermined manner;
(e) if the second sensor is a chemical sensor that has a different sensitivity to chemical analytes than the first sensor, determining whether the presence or concentration of at least one trace metabolite in the exhaled air indicates whether the user is exhaling air through his mouth or through his nose.

10. A computer program element comprising computer code that, when executed in a processor of a determination unit at a remote server (250), the determination unit being in communication with a portable electronic device according to claim 4, carries out the method of claim 9.

## Patentansprüche

1. Tragbares elektronisches Gerat (1), welches dazu ausgebildet ist, mindestens einen chemischen Analyten in Luft, die aus dem Mund eines Benutzers exhaliert wird, zu detektieren, aufweisend:
eine Steuervorrichtung (201);
mindestens einen Sensor (226), wobei der erste Sensor (226) auf mindestens einen chemischen Analyten empfindlich ist, wobei der erste Sensor (226) mit der Steuervorrichtung (201) verbunden ist;
mindestens einen zweiten Sensor (211-225), welcher mit der Steuervorrichtung (221) verbunden ist;
und mindestens eine Ausgabevorrichtung (230-236), die mit der Steuervorrichtung verbunden ist;
wobei die Steuervorrichtung dazu ausgebildet ist, die folgenden Schritte auszuführen:
Ausführen einer Messung mit dem ersten Sensor (226);
Ermitteln, ob das tragbare elektronische Gerät (1) während der Messung korrekt gehandhabt wurde; und
falls das tragbare elektronische Gerät (1) nicht korrekt gehandhabt wurde, Betreiben der Ausgabevorrichtung (230-236) zur Ausgabe von Informationen an einen Benutzer des tragbaren elektronischen Geräts (1),
**dadurch gekennzeichnet, dass**
der mindestens eine zweite Sensor (211-225) ein Feuchtigkeitssensor, ein CO₂-Sensor, ein Mikrofon, ein Beschleunigungssensor, ein Gyroskop oder ein zweiter chemischer Sensor mit einer anderen Empfindlichkeit auf chemische Analyten als der erste Sensor ist, und dass
die Ermittlung, ob das tragbare elektronische Gerät (1) während der Messung korrekt gehandhabt wurde, auf einem der folgenden Kriterien beruht:
(a) falls der zweite Sensor ein Feuchtigkeitssensor ist, ermitteln, ob die mit dem Feuchtigkeitssensor gemessene Feuchtigkeit für eine Zeitdauer einen Schwellwert überschreitet, um anzuzeigen, dass der Benutzer Luft auf eine vorbestimmte Weise exhaliert;
(b) falls der zweite Sensor ein CO₂-Sensor ist, ermitteln, ob die CO₂-Konzentration, die mit dem CO₂-Sensor gemessen wird, innerhalb einer Zeitdauer über einen Schwellwert steigt, um anzuzeigen, dass der Benutzer Luft in einer bestimmten Weise exhaliert;
(c) falls der zweite Sensor ein Mikrofon ist, ermitteln, ob mit dem Mikrofon aufgezeichnete Schallsignale einem charakteristischen Geräuschmuster entsprechen, um anzuzeigen, dass der Benutzer Luft in einer bestimmten Weise exhaliert;
(d) falls der zweite Sensor ein Beschleunigungssensor oder ein Gyroskop ist, ermitteln, ob eine gemessene Beschleunigung oder Orientierung des tragbaren elektronischen Geräts anzeigt, dass das tragbare elektronische Gerät schnellen linearen oder rotatorischen Bewegungen ausgesetzt wird oder dass das tragbare elektronische Gerät in eine unerwünschte Richtung zeigt, um anzuzeigen, ob der Benutzer das tragbare elektronische Gerät auf eine bestimmt Weise hält;
(e) falls der zweite Sensor ein chemischer Sensor mit einer anderen Empfindlichkeit auf chemische Analyten als der erste Sensor ist, ermitteln, ob die Anwesenheit oder Konzentration mindestens eines Spuren-Metaboliten in der exhalierten Luft anzeigt, ob der Benutzer Luft durch seinen Mund oder seine Nase exhaliert.

2. Tragbares elektronisches Gerät nach Anspruch 1, wobei die Information Anweisungen an den Benutzer umfasst, das tragbare elektronische Gerät (1) auf eine bestimmte Weise handzuhaben.

3. Tragbares elektronisches Gerät nach einem der vorhergehenden Ansprüche, wobei das tragbare elektronische Gerät (1) mindestens zwei zweite Sensoren umfasst, wobei jeder der zweiten Sensoren betreibbar ist, um einen anderen Parameter zu bestimmen, und wobei die Steuervorrichtung dazu ausgebildet ist, mindestens zwei von verschiedenen zweiten Sensoren gemessene Parameter zu kombinieren, wenn sie ermittelt, ob das tragbare elektronische Gerät (1) korrekt gehandhabt wurde.

4. Tragbares elektronisches Gerät nach einem der vorhergehenden Ansprüche, wobei das tragbare elektronische Gerät (1) mindestens ein Kommunikationsmodul (240, 241, 242) aufweist, wobei die Steuervorrichtung (201) dazu ausgebildet ist, Sensordaten über das mindestens eine Kommunikationsmodul an einen Remote-Server (250) zu senden, und in Antwort darauf von dem Remote-Server über das mindestens eine Kommunikationsmodul Statusdaten zu empfangen, die indikativ für die Handhabung des tragbaren elektronischen Geräts sind.

5. Verfahren zum Betrieb eines tragbaren elektronischen Geräts (1), welches mindestens einen ersten Sensor (226) aufweist, wobei der erste Sensor (226) auf mindestens einen chemischen Analyten empfindlich ist, und welches mindestens einen zweiten Sensor aufweist, wobei das Verfahren aufweist:
Ausführen einer Messung mit dem ersten Sensor (226);
Ermitteln, ob das tragbare elektronische Gerät (1) während der Messung korrekt gehandhabt wurde; und
falls das tragbare elektronische Gerät (1) nicht korrekt gehandhabt wurde, Betreiben der Ausgabevorrichtung (230-236) zur Ausgabe von Informationen an einen Benutzer des tragbaren elektronischen Geräts (1),
**dadurch gekennzeichnet, dass**
der mindestens eine zweite Sensor (211-225) ein Feuchtigkeitssensor, ein CO₂-Sensor, ein Mikrofon, ein Beschleunigungssensor, ein Gyroskop oder ein zweiter chemischer Sensor mit einer anderen Empfindlichkeit auf chemische Analyten als der erste Sensor ist, und dass
die Ermittlung, ob das tragbare elektronische Gerät (1) während der Messung korrekt gehandhabt wurde, auf einem der folgenden Kriterien beruht:
(a) falls der zweite Sensor ein Feuchtigkeitssensor ist, ermitteln, ob die mit dem Feuchtigkeitssensor gemessene Feuchtigkeit für eine Zeitdauer einen Schwellwert überschreitet, um anzuzeigen, dass der Benutzer Luft auf eine vorbestimmte Weise exhaliert;
(b) falls der zweite Sensor ein CO₂-Sensor ist, ermitteln, ob die CO₂-Konzentration, die mit dem CO₂-Sensor gemessen wird, innerhalb einer Zeitdauer über einen Schwellwert steigt, um anzuzeigen, dass der Benutzer Luft in einer bestimmten Weise exhaliert;
(c) falls der zweite Sensor ein Mikrofon ist, ermitteln, ob mit dem Mikrofon aufgezeichnete Schallsignale einem charakteristischen Geräuschmuster entsprechen, um anzuzeigen, dass der Benutzer Luft in einer bestimmten Weise exhaliert;
(d) falls der zweite Sensor ein Beschleunigungssensor oder ein Gyroskop ist, ermitteln, ob eine gemessene Beschleunigung oder Orientierung des tragbaren elektronischen Geräts anzeigt, dass das tragbare elektronische Gerät schnellen linearen oder rotatorischen Bewegungen ausgesetzt wird oder dass das tragbare elektronische Gerät in eine unerwünschte Richtung zeigt, um anzuzeigen, ob der Benutzer das tragbare elektronische Gerät auf eine bestimmt Weise hält;
(e) falls der zweite Sensor ein chemischer Sensor mit einer anderen Empfindlichkeit auf chemische Analyten als der erste Sensor ist, ermitteln, ob die Anwesenheit oder Konzentration mindestens eines Spuren-Metaboliten in der exhalierten Luft anzeigt, ob der Benutzer Luft durch seinen Mund oder seine Nase exhaliert.

6. Verfahren nach Anspruch 5, wobei die Information Anweisungen an den Benutzer umfasst, das tragbare elektronische Gerät (1) auf eine bestimmte Weise handzuhaben.

7. Verfahren nach Anspruch 5 oder 6, umfassend das Senden von Sensordaten an einen Remote-Server (250) und, in Antwort darauf, das Empfangen von Statusdaten vom Remote-Server, die indikativ für die Handhabung des tragbaren elektronischen Geräts sind.

8. Computerprogrammelement umfassend Computercode, welcher, wenn er in einer Steuervorrichtung (221) eines tragbaren elektronischen Geräts (1), aufweisend mindestens einen ersten Sensor (226), wobei der erste Sensor (226) auf mindestens einen chemischen Analyten empfindlich ist, ausgeführt wird, das Verfahren nach einem der Ansprüche 5-7 ausführt.

9. Verfahren zum Betrieb einer Auswerteeinheit an einem Remote-Server (250), wobei die Auswerteeinheit in Kommunikation mit einem tragbaren elektronischen Gerät nach Anspruch 4 steht, wobei das Verfahren aufweist:
Empfangen von Sensordaten vom tragbaren elektronischen Gerät (1) am Remote-Server (250), wobei die Sensordaten Daten umfassen, die aus Messungen mit dem ersten und zweiten Sensor abgeleitet wurden;
Herleiten von Statusdaten, die indikativ für die Handhabung des tragbaren elektronischen Geräts (1) sind, und
Senden der Statusdaten an das tragbare elektronische Gerät (1), wobei die Statusdaten basierend auf einem der folgenden Kriterien hergeleitet werden:
(a) falls der zweite Sensor ein Feuchtigkeitssensor ist, ermitteln, ob die mit dem Feuchtigkeitssensor gemessene Feuchtigkeit für eine Zeitdauer einen Schwellwert überschreitet, um anzuzeigen, dass der Benutzer Luft auf eine vorbestimmte Weise exhaliert;
(b) falls der zweite Sensor ein CO₂-Sensor ist, ermitteln, ob die CO₂-Konzentration, die mit dem CO₂-Sensor gemessen wird, innerhalb einer Zeitdauer über einen Schwellwert steigt, um anzuzeigen, dass der Benutzer Luft in einer bestimmten Weise exhaliert;
(c) falls der zweite Sensor ein Mikrofon ist, ermitteln, ob mit dem Mikrofon aufgezeichnete Schallsignale einem charakteristischen Geräuschmuster entsprechen, um anzuzeigen, dass der Benutzer Luft in einer bestimmten Weise exhaliert;
(d) falls der zweite Sensor ein Beschleunigungssensor oder ein Gyroskop ist, ermitteln, ob eine gemessene Beschleunigung oder Orientierung des tragbaren elektronischen Geräts anzeigt, dass das tragbare elektronische Gerät schnellen linearen oder rotatorischen Bewegungen ausgesetzt wird oder dass das tragbare elektronische Gerät in eine unerwünschte Richtung zeigt, um anzuzeigen, ob der Benutzer das tragbare elektronische Gerät auf eine bestimmt Weise hält;
(e) falls der zweite Sensor ein chemischer Sensor mit einer anderen Empfindlichkeit auf chemische Analyten als der erste Sensor ist, ermitteln, ob die Anwesenheit oder Konzentration mindestens eines Spuren-Metaboliten in der exhalierten Luft anzeigt, ob der Benutzer Luft durch seinen Mund oder seine Nase exhaliert.

10. Computerprogrammelement aufweisend Computercode, welcher, wenn er in einem Prozessor einer Auswerteeinheit an einen Remote-Server (250) ausgeführt wird, wobei die Auswerteeinheit in Kommunikation mit einem tragbaren elektronischen Gerät nach Anspruch 4 steht, das Verfahren nach Anspruch 9 ausführt.

## Revendications

1. Un dispositif électronique portable (1) configuré pour détecter au moins un analyte chimique dans de l'air exhalé depuis la bouche d'un utilisateur, comprenant :
un dispositif de contrôle (201) ;
au moins un premier capteur (226), le premier capteur (226) étant sensible à au moins un analyte chimique, le premier capteur (226) étant couplé au dispositif de contrôle (201) ;
au moins un deuxième capteur (211-225) couplé au dispositif de contrôle (201) ; et
au moins un dispositif de sortie (230-236) couplé au dispositif de contrôle (201) ;
dans lequel le dispositif de contrôle est configuré pour effectuer les étapes suivantes :
effectuer une mesure à l'aide du premier capteur (226) ;
déterminer si le dispositif électronique portable (1) a été manipulé de manière adéquate durant la mesure ; et
si le dispositif électronique portable (1) n'a pas été manipulé de manière adéquate, opérer le dispositif de sortie (230-236) pour présenter de l'information à un utilisateur du dispositif électronique portable (1),
**caractérisé en ce que**
l'au moins un deuxième capteur (211-225) est un capteur d'humidité, un capteur à CO₂, un microphone, un capteur d'accélération, un gyroscope, ou un deuxième capteur chimique ayant une sensibilité aux analytes chimiques différente à celle du premier capteur, et **en ce que**
la détermination si le dispositif électronique portable (1) a été manipulé de manière adéquate durant la mesure est basée sur au moins un des suivants :
(a) si le deuxième capteur est un capteur d'humidité, déterminer si l'humidité mesurée par le capteur d'humidité excède un seuil pour une période de temps de sorte à indiquer que l'utilisateur exhale l'air d'une manière prédéterminée,
(b) si le deuxième capteur est un capteur CO₂, déterminer si la concentration de CO₂ mesurée par le capteur de CO₂ augmente au-delà d'un seuil dans une période de temps de sorte à indiquer que l'utilisateur exhale l'air d'une manière prédéterminée ;
(c) si le deuxième capteur est un microphone, déterminer si le signal sonore enregistré par le microphone correspond à un motif de bruit caractéristique de sorte à indiquer que l'utilisateur exhale l'air d'une manière prédéterminée ;
(d) si le deuxième capteur est un capteur d'accélération ou un gyroscope, déterminer si l'accélération mesurée ou l'orientation du dispositif électronique portable indique que le dispositif électronique portable est soumis à des mouvements rapides linéaires ou rotationnels, ou que le dispositif électronique portable pointe dans une direction non-désirée, de sorte à indiquer si l'utilisateur tient le dispositif électronique portable d'une manière prédéterminée ;
(e) si le deuxième capteur est un capteur chimique qui a une sensibilité aux analystes chimiques différente à celle du premier capteur, déterminer si la présence ou la concentration d'au moins un métabolite de trace dans l'air exhalé indique si l'utilisateur exhale l'air à travers sa bouche ou à travers son nez.

2. Le dispositif électronique portable selon la revendication 1, dans lequel l'information à l'utilisateur inclut des instructions pour manipuler le dispositif électronique portable (1) d'une manière spécifique.

3. Le dispositif électronique portable selon l'une quelconque des revendications précédentes, dans lequel le dispositif électronique portable (1) comprend au moins deux deuxièmes capteurs, chacun des deuxièmes capteurs étant opérable pour mesurer un paramètre différent et dans lequel le dispositif de contrôle est configuré pour combiner au moins deux paramètres mesurés par de différents capteurs lors de la détermination si le dispositif électronique portable (1) a été manipulé de manière correcte.

4. Le dispositif électronique portable selon l'une quelconque des revendications précédentes, dans lequel le dispositif électronique portable comprend au moins un module de communication (240, 241, 242) et dans lequel le dispositif de contrôle (201) est configuré pour envoyer des données de capteur à un serveur distant (250) à travers au l'au moins un module de communication et pour recevoir, en réponse, des données d'état indicatives de la manipulation du dispositif électronique portable, dudit serveur distant à travers l'au moins un module communication.

5. Un procédé pour opérer un dispositif électronique portable (1) comprenant au moins un premier capteur (226), le premier capteur (226) étant sensible à au moins un analyte chimique, et au moins un deuxième capteur, le procédé comprenant :
effectuer une mesure à l'aide du premier capteur (226) ;
déterminer si le dispositif électronique portable (1) a été manipulé de manière adéquate durant la mesure ; et
si le dispositif électronique portable (1) n'a pas été manipulé de manière adéquate, fournir de l'information à un utilisateur du dispositif électronique portable (1),
**caractérisé en ce que**
l'au moins un deuxième capteur (211-225) est un capteur d'humidité, un capteur à CO2, un microphone, un capteur d'accélération, un gyroscope, ou un deuxième capteur chimique qui a une sensibilité aux analytes chimiques différente à celle du premier capteur, et **en ce que**
la détermination si le dispositif électronique portable (1) a été manipulé de manière adéquate durant la mesure est basée sur au moins un des suivants :
(a) si le deuxième capteur est un capteur d'humidité, déterminer si l'humidité mesurée par le capteur d'humidité excède un seuil pour une période de temps de sorte à indiquer que l'utilisateur exhale l'air d'une manière prédéterminée,
(b) si le deuxième capteur est un capteur à CO2, déterminer si la concentration de CO2 mesurée par le capteur à CO2 augmente au-delà d'un seuil dans une période de temps de sorte à indiquer que l'utilisateur exhale l'air d'une manière prédéterminée ;
(c) si le deuxième capteur est un microphone, déterminer si les signaux sonores enregistrés par le microphone correspondent à un motif de bruit caractéristique de sorte à indiquer que l'utilisateur exhale l'air d'une manière prédéterminée ;
(d) si le deuxième capteur est un capteur d'accélération ou un gyroscope, déterminer si l'accélération mesurée ou l'orientation du dispositif électronique portable indique que le dispositif électronique portable est soumis à des mouvements rapides linéaires ou rotationnels, ou que le dispositif électronique portable pointe dans une direction non-désirée, de sorte à indiquer si l'utilisateur tient le dispositif électronique portable d'une manière prédéterminée ;
(e) si le deuxième capteur est un capteur chimique qui a une sensibilité aux analytes chimiques différente à celle du premier capteur, déterminer si la présence ou la concentration d'au moins un métabolite de trace dans l'air exhalé indique si l'utilisateur exhale l'air à travers sa bouche ou à travers son nez.

6. Le dispositif selon la revendication 5, dans lequel l'information inclut des instructions à l'utilisateur pour manipuler le dispositif électronique portable d'une manière spécifique.

7. Le dispositif selon la revendication 5 ou 6, comprenant envoyer des données de capteur à un serveur distant (250) et recevoir, en réponse, des données d'état indicatives de la manipulation du dispositif électronique portable à partir dudit serveur distant.

8. Un élément de logiciel d'ordinateur comprenant du code informatique qui, lorsqu'il est exécuté dans un dispositif de contrôle (201) d'un dispositif électronique portable (1) comprenant au moins un premier capteur (226), le premier capteur (226) étant sensible à au moins un analyte chimique, effectue la méthode selon l'une quelconque des revendications 5 à 7.

9. Un procédé pour opérer une unité de détermination sur un serveur distant (250), l'unité de détermination étant en communication avec un dispositif électronique portable selon la revendication 4, le procédé comprenant :
recevoir des données de capteur à travers un dispositif électronique portable (1) au niveau du serveur distant (250), les données de capteur comprenant des données dérivées des mesures avec le premier et deuxième capteur ;
dériver des données d'état indicatives de la manipulation du dispositif électronique portable (1), et
envoyer lesdites données d'état au dispositif électronique portable (1),
dans lequel les données d'état sont dérivées à travers au moins un des suivants :
(a) si le deuxième capteur est un capteur d'humidité, déterminer si l'humidité mesurée par le capteur d'humidité excède un seuil pour une période de temps de sorte à indiquer que l'utilisateur exhale l'air d'une manière prédéterminée,
(b) si le deuxième capteur est un capteur à CO2, déterminer si la concentration en CO2 mesurée par le capteur à CO2 augmente au-delà d'un seuil dans une période de temps de sorte à indiquer que l'utilisateur exhale l'air d'une manière prédéterminée ;
(c) si le deuxième capteur est un microphone, déterminer si les signaux sonores enregistrés par le microphone correspondent à un motif de bruit caractéristique de sorte à indiquer que l'utilisateur exhale l'air d'une manière prédéterminée ;
(d) si le deuxième capteur est un capteur d'accélération ou un gyroscope, déterminer si l'accélération ou l'orientation mesurée du dispositif électronique portable indique que le dispositif électronique portable est soumis à des mouvements rapides linéaires ou rotationnels, ou que le dispositif électronique portable pointe dans une direction non-désirée, de sorte à indiquer si l'utilisateur tient le dispositif électronique portable d'une manière prédéterminée ;
(e) si le deuxième capteur est un capteur chimique qui a une sensibilité aux analytes chimiques différente à celle du premier capteur, déterminer si la présence ou la concentration d'au moins un métabolite de trace dans l'air exhalé indique si l'utilisateur exhale l'air à travers sa bouche ou à travers son nez.

10. Un élément de logiciel d'ordinateur comprenant du code informatique qui, lorsqu'il est exécuté dans un processeur d'une unité de détermination au niveau d'un serveur distant (250), l'unité de détermination étant en communication avec le dispositif électronique portable selon la revendication 4 effectue la méthode de la revendication 9.
